Europäisches Patentamt

European Patent Office

Office européen des brevets

(19)

(11) Veröffentlichungsnummer: **0 195 124**
**A2**

(12) # EUROPÄISCHE PATENTANMELDUNG

(21) Anmeldenummer: 85115505.1

(22) Anmeldetag: 06.12.85

(51) Int. Cl.⁴: **B 01 D 11/04**

(30) Priorität: 15.02.85 DE 3505241

(43) Veröffentlichungstag der Anmeldung:
24.09.86 Patentblatt 86/39

(84) Benannte Vertragsstaaten:
BE FR GB IT LU NL

(71) Anmelder: PIERBURG GmbH & Co. KG
Leuschstrasse 1
D-4040 Neuss 1(DE)

(72) Erfinder: Heckershoff, Herbert
Kranichstrasse 2
D-4044 Kaarst(DE)

(74) Vertreter: Bergen, Klaus, Dipl.-Ing. et al,
Patentanwälte Dr.-Ing. Reimar König Dipl.-Ing. Klaus
Bergen Wilhelm-Tell-Strasse 14 Postfach 260162
D-4000 Düsseldorf 1(DE)

(54) Verfahren und Vorrichtung zum Rückgewinnen des Lösungsmittels beim Messen des Ölgehaltes von Wasser.

(57) Bei einem Verfahren zum Rückgewinnen des beim Messen des Ölgehaltes von Wasser mit einer Menge ölbeladenem Probenwasser in einem Extraktor (32) extrahiertem organischen Lösungsmittels, läßt sich nach der Messung der Aufbereitungs aufwand zum Abtrennen des Lösungsmittels von dem Öl und erneuten Einbringen in den Kreislauf dann verringern, wenn eine im Extraktor (32) nach dem Entnehmen einer für das Messen erforderlichen Mebprobenmenge verbliebene Rest-Flüssigkeitsmenge aus mit dem Lösungsmittel extrahiertem, ölbeladenem Probenwasser in einer anschliebenden Trennstufe ebenso wie die Meßprobenmenge vom Wasseranteil befreit wird, bevor sie, wie auch die vom Wasser befreite Meßprobenmenge, einem Sammelbehälter (51) für wasserfreie, ölbeladene Lösungsmittel-Flüssigkeit zugeführt wird. Ein dem Extraktor (32) nachgeordnetes Trennfilter (43) kann hierzu eine abgetrenntes Wasser führende und eine ölbeladene Lösungsmittel-Flüssigkeit führende Leitung (34 bzw. 44, 50) aufweisen, von denen die Lösungsmittel-Flüssigkeitsleitung (44, 50) in einen separaten Flüssigkeits-Sammelbehälter (51) mündet.

Pierburg GmbH & Co. KG, Leuschstraße 1,
=========================================

4040 Neuss 1
=============

"Verfahren und Vorrichtung zum Rückgewinnen des
Lösungsmittels beim Messen des Ölgehaltes von Wasser"

Die Erfindung betrifft ein Verfahren zum Rückgewinnen des beim Messen des Ölgehaltes von Wasser mit einer Menge ölbeladenem Probenwasser in einem Extraktor extrahierten organischen Lösungsmittels, sowie eine Vorrichtung zum Durchführen des Verfahrens.

Bei einer mit der älteren deutschen Patentanmeldung P 34 17 454.0 vorgeschlagenen Vorrichtung, die beispielsweise zum Überwachen öffentlicher Gewässer dienen kann, die der Gefahr einer unzuträglichen Belastung mit Kohlenwasserstoffen ausgesetzt sind, wie z.B. die Entwässerungskanäle von Flugplätzen, oder der Überwachung der Kreislaufflüssigkeiten von Öl/Wasserwärmeaustauschern und der Kontrolle bei der Schneidölaufbereitung dienen und in Fabriken, Flughäfen, Bahnhöfen, Tankstellen, Waschanlagen oder Laborbetrieben eingesetzt werden kann, wird die in einem Extraktor extrahierte Flüssigkeit aus einem Lösungsmittel und einem ölbeladenen Probenwasser nach dem Extrahieren aufgeteilt. Ein bestimmter Anteil der innig miteinander vermischten Flüssigkeit gelangt über eine Meßleitung in einen Trennfilter, in dem das Wasser aus der Probenmenge gefiltert und einem Sammelbehälter zugeführt wird. Die vom Wasser befreite Probenmenge gelangt dann vom Filter über eine Leitung in eine Küvette eines Infrarot-Meßgerätes, die nach mehrmaligem Spülen mit der Probenmenge zum Ermitteln des Meßwertes gefüllt wird.

Nach dem Messen wird die ölbeladene Probenmenge in einem Behälter gesammelt, der auch das zuvor im Filter abgetrennte Wasser und die im Extraktor verbliebene Rest-Flüssigkeitsmenge aus mit dem Lösungsmittel extrahiertem, ölbeladenem Probenwasser aufnimmt. Das bedeutet, daß in den Sammelbehälter sowohl vom Wasser befreite Lösungs-/Öl-Flüssigkeitsmengen, als auch gefiltertes Wasser, als auch mit dem Lösungsmittel extrahiertes ölbeladenes Probenwasser zusammengeführt werden. Das Aufbereiten bzw. Wiedergewinnen des teuren Lösungsmittels, wie Tetrachlorkohlenstoff oder Trichlortrifluorethan, muß - um es dem Kreislauf erneut zuführen zu können - in zwei Stufen vorgenommen werden. Es wird nämlich zunächst das Wasser-/Lösungsmittel-/Ölgemisch über ein Koagulierelement vom Wasser befreit und danach das Lösungsmittel des Lösungsmittel-/Ölgemisches über Aktivkohle bzw. mittels Destillation zurückgewonnen.

Der Erfindung liegt die Aufgabe zugrunde, den Aufwand beim Aufbereiten bzw. Rückgewinnen des Lösungsmittels aus dem Flüssigkeitsgemisch zu verringern und insbesondere mit lediglich einem einstufigen Aufbereitungs- bzw. Trennvorgang auszukommen.

Diese Aufgabe wird nach der Erfindung verfahrensmäßig dadurch gelöst, daß eine im Extraktor nach dem Entnehmen einer für das Messen erforderlichen Meßprobenmenge verbliebene Rest-Flüssigkeitsmenge aus mit dem Lösungsmittel extrahiertem, ölbeladenem Probenwasser in einer Trennstufe ebenso wie die Meßprobenmenge vom Wasseranteil befreit wird, bevor sie, wie auch die vom Wasser befreite Meßprobenmenge, einem Sammelbehälter für wasserfreie, ölbeladene Lösungsmittel-Flüssigkeit zugeführt wird. Hierbei wird davon ausgegangen, daß die zum Spülen und Messen dem Extraktor entnommene Meßprobenmenge durch das der Messung vorgeschaltete Filtern bereits vom Wasser befreit worden ist, was eine Grundbedingung für eine einwandfreie Messung ist, und der Lösungsmittel-Anteil der Probenmenge ca. 50 bis 60% des insgesamt eingebrachten Lösungsmittels beträgt. Der Rest des Lösungsmittels, d.h. ca. 40 bis 50% der Gesamtmenge, befindet sich noch im Extraktor, da zur Messung nicht das gesamte Flüssigkeitsvolumen des Extraktors benötigt wird. Dieses restliche Wasser-, Öl- und Lösungsmittelgemisch wird nun nicht mehr sofort in einen Sammelbehälter abgelassen, der auch das wasserfreie Lösungsmittel-/Ölgemisch aufnimmt, sondern zunächst ebenfalls von seinem Wasseranteil befreit. Auf diese Weise erreicht den Sammelbehälter ausschließlich eine wasserfreie Flüssigkeit, so daß ein einstufiges Aufbereiten, beispielsweise durch Adsorption an Aktivkohle, zum Rückgewinnen des Lösungsmittels ausreicht. Dadurch lassen sich bis ca. 95% des eingesetzten Lösungsmittels als wasserfreies Öl-/Lösungsmittelgemisch rückgewinnen.

Um die Belastung des nachgeschalteten Filters herabzusetzen, läßt sich aus dem Extraktor eine untere Schicht der Rest-Flüssigkeitsmenge aus sich gegenüber dem Wasseranteil absetzendem, ölbeladenem Lösungsmittel über die Trennstufe in den Sammelbehälter für wasserfreie Lösungsmittel-Flüssigkeit und eine obere, weitestgehend lösungsmittelfreie Wasserschicht direkt in den abgetrenntes Wasser sammelnden Behälter leiten. Im Extraktor bilden sich aus der Rest-Flüssigkeitsmenge wegen des gegenüber dem Wasser schwereren Lösungsmittels nämlich zwei Flüssigkeitsschichten; das ölbeladene Lösungsmittel setzt sich mit einer relativ klaren Trennlinie am Boden des Extraktorgefäßes ab. Hierbei durchläuft im Anschluß an die Meßprobenmenge nicht mehr die gesamte, im Extraktor verbliebene Rest-Flüssigkeitsmenge das Trennfilter, sondern nur noch das abgesetzte, weitestgehend wasserfreie, ölbeladene Lösungsmittel, so daß sich etwaige Wasserbestandteile sicher daraus entfernen lassen.

Das Separieren und getrennte Abführen der Flüssigkeiten läßt sich dadurch erreichen, daß das an den Extraktor angeschlossene, nachgeordnete Trennfilter eine abgetrenntes Wasser führende und eine ölbeladene Lösungsmittel-Flüssigkeit führende Leitung aufweist, von denen die Lösungsmittel-Flüssigkeits-Leitung in einen separaten Flüssigkeits-Sammelbehälter mündet. Die Ablaufsteuerung des Gerätes läßt sich so ändern, daß nach dem eigentlichen Messen auch der Rest des Extraktorinhalts über das Trennfilter zum Abtrennen des Wassers geführt wird; das abgetrennte Lösungsmittel-/Ölgemisch gelangt danach nicht mehr mit Wasser in Berührung.

Hierzu kann eine Entleerungsleitung des Extraktors vor dem Trennfilter an eine mit dem Trennfilter verbundene Meßleitung des Extraktors angeschlossen sein und die dem Filter nachgeschaltete Leitung der ölbeladenen Lösungsmittel-Flüssigkeit einerseits zu dem Meßgerät und andererseits mit einem sich einem in der Leitung angeordneten Ablaufventil anschließenden Leitungsfortsatz in den Sammelbehälter reichen. Damit läßt sich, wenn eine Entleerungs- und eine Meßleitung vorhanden sind, was für einen schnellen Prozeßablauf sorgt, der Bau- bzw. Leitungsaufwand reduzieren, da lediglich eine in das Filter hinein- und aus diesem herausführende Leitung ausreicht.

Die Erfindung wird nachfolgend anhand eines schematisch als Fließschema dargestellten, im Rahmen der Erfindung bevorzugten Prozeßgerätes näher erläutert.

Die von einem üblichen Analyse- bzw. Laborgerät 1 separate Dosiereinheit 2, was durch die gestrichelte Trennlinie 3 verdeutlicht wird, besteht im wesentlichen aus einem Dosierbehälter 4 für das Lösungsmittel und einem Dosierbehälter 5 für das Probenwasser, wobei in den Behältern 4, 5 jeweils zylindrische Aufnahmekammern 6, 7 für die Flüssigkeiten vorgesehen sind. Jedem Dosierbehälter 4, 5 ist eine Entlüftung 8 und im Bereich der Überlaufränder 9, 12 der oben offenen Aufnahmekammern 6,7 ein Näherungsschalter 13 sowie je ein Verdrängungskegel 14 zugeordnet, der sich in die Aufnahmekammer eintauchen läßt.

An jede Aufnahmekammer 6, 7 ist eine Dosierleitung 15, 16 mit einem darin angeordneten Mehrwegeventil 17, 18 angeschlossen. Von den Mehrwegeventilen 17, 18 gehen Zweigleitungen 19, 22 mit daran angeschlossenen Pumpen 23, 24 ab. Während die Pumpe 24 das Probenwasser über das Drei-Wege-Ventil 18 aus einem beliebigen, nicht dargestellten Behälter in den Dosierbehälter 5 fördert, wo es nach dem Überlaufen mittels des Ablaufs 25 entfernt wird, fördert die Pumpe 23 das Lösungsmittel in einem geschlossenen Kreislauf. Dazu reicht die Zweigleitung 19 bis in einen Vorratsbehälter 26, wie auch eine Rücklaufleitung 27 das am Überlaufrand 9 überströmende Lösungsmittel in den Behälter 26 zurückführt.

Die Dosierleitungen 15, 16 der Dosierbehälter 4, 5 lassen sich mittels Anschlüssen bzw. Anschlußleitungen 28, 29 mit einem Extraktor 32 des Laborgerätes 1 verbinden. Eine motorbetriebene Taumelscheibe 33 vermischt die beiden in dem Extraktionsgefäß zusammentreffenden Förderströme des Lösungsmittels und des Probenwassers innig miteinander, die sich darin völlig extrahieren lassen. Aus dem Extraktor 32 führt neben einer Meßleitung 34 geringeren Durchmessers auch eine Entleerungsleitung 35 mit einem größeren Durchmesser heraus.

Sowohl in der Entleerungsleitung 35 als auch in der Meßleitung 34 sind zwei steuerbare Ablaufventile 37, 40; 38, 39 angeordnet, von denen das Ablaufventil 38 bei abgesperrtem Ventil 39 über eine Leitung 42 die extrahierte Flüssigkeit einer Meßprobenmenge zu einem Trennfilter 43 leitet. Im Trennfilter 43 wird vor dem Messen die Öllösung von dem Wasser abgetrennt, die dann zur Meßwertermittlung über eine sich dem Filter 43 anschließende Verbindungsleitung 44 zu einem Infrarot-Strahlenanalysator 45 gelangt. Nach dem Messen strömt die Öllösung über ein in der Leitung 44 angeordnetes Ablaufventil 46 und einen sich dem Ablaufventil anschließenden Leitungsfortsatz 50 in einen Sammelbehälter 51 mit Ablauf 54. Der Behälter 51 und eine nicht dargestellte Entsorgungs- und/oder Trenn- und Aufbereitungseinrichtung lassen sich ebenfalls als separater Bausatz ausbilden und unterhalb der gestrichelten Trennlinie 48 mit der Dosiereinheit 2 und dem Laborgerät 1 zu einer kompakten Einheit zusammensetzen.

Die gegenüber der Meßleitung 34 im Durchmesser größere Entleerungsleitung 35 vereint sich einerseits hinter dem Ablaufventil 37 über einen Leitungsabschnitt 55 mit der von der Meßleitung 34 zum Trennfilter 43 führenden Leitung 42, so daß auch das hierüber in den Trennfilter gelangende Flüssigkeitsgemisch vom Wasser befreit wird; andererseits führen die Leitungen 34, 35 bis in einen separaten Auffangbehälter 47 mit Ablaß 49, wobei das sich im Filter 43 ansammelnde Wasser bei geöffnetem Magnetventil 39 über die Leitung 34 abgeführt wird. Das Lösungsmittel-/Ölgemisch des entleerten Extraktors gelangt - wie zuvor schon die Probenmenge - über die Leitung 44, das Ablaufventil 46 und den Leitungsfortsatz 50 ebenfalls in den Sammelbehälter 51. Die dem Sammelbehälter 51 an dem Ablauf 54 zu entnehmende Flüssigkeit läßt sich zum Rückgewinnen des Lösungsmittels in einem schematisch dargestellten Kohlefilter 52 aufbereiten. Um einen kontinuierlichen Kreislauf und eine automatische Betriebsweise auch über längere Zeiträume zu ermöglichen, kann das Kohlefilter 52 - wie strichpunktiert dargestellt - mittels einer Versorgungsleitung 53 direkt an den Vorratsbehälter 26 für das Lösungsmittel angeschlossen werden. Wenn der von der Entleerungsleitung 35 zu dem Trennfilter 43 führende Leitungsabschnitt 55 vorhanden ist, läßt sich das Gerät wunschweise auch ohne besondere Meßleitung 34 betreiben.

Um gegebenenfalls nicht den gesamten Inhalt des Extraktors 32 über das Trennfilter 43 in den Sammelbehälter 51 für wasserfreies Lösungsmittel leiten zu müssen, sondern nur die Meßprobenmenge und das sich im Extraktor am Boden absetzende, ölbeladene Lösungsmittel, werden nach der Meßprobennahme zunächst nur die in den Leitungen 34, 35 oberhalb der zum Trennfilter 43 führenden Zweigleitungen 42, 55 angeordneten Magnetventile 37, 38 geöffnet und zwar solange, bis die untere ölbeladene Lösungsmittelschicht abgeflossen ist. Erst danach werden auch die unteren Magnetventile 39, 40 geöffnet, so daß das weitestgehend lösungsmittelfreie Wasser aus dem Extraktor auf direktem Weg in den Behälter 47 strömt.

2 br

- 8 -

Pierburg GmbH & Co. KG, Leuschstraße 1,
=========================================

4040 Neuss 1
==============

"Verfahren und Vorrichtung zum Rückgewinnen des
Lösungsmittels beim Messen des Ölgehaltes von Wasser"

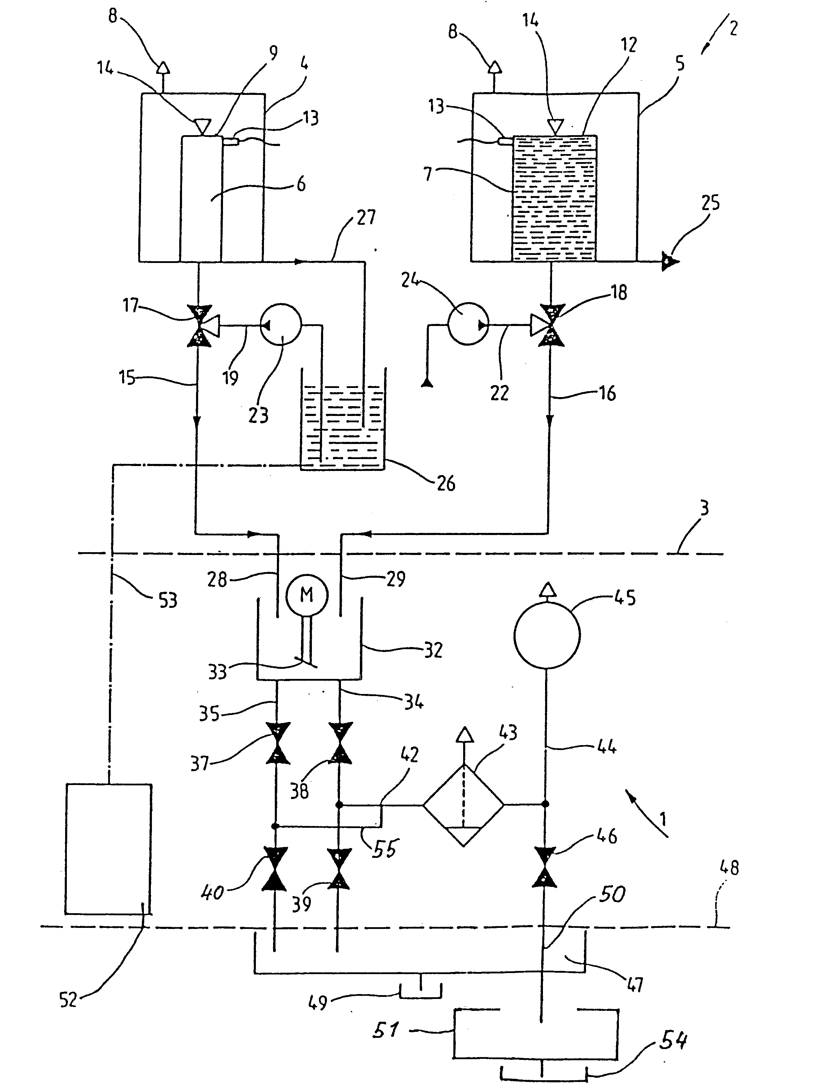

Patentansprüche:

1. Verfahren zum Rückgewinnen des beim Messen des Ölgehaltes von Wasser mit einer Menge ölbeladenem Probenwasser in einem Extraktor extrahiertem organischen Lösungsmittels, dadurch gekennzeichnet, daß eine im Extraktor nach dem Entnehmen einer für das Messen erforderlichen Meßprobenmenge verbliebene Rest-Flüssigkeitsmenge aus mit dem Lösungsmittel extrahiertem, ölbeladenem Probenwasser in einer Trennstufe ebenso wie die Meßprobenmenge vom Wasseranteil befreit wird, bevor sie, wie auch die vom Wasser befreite Meßprobenmenge, einem Sammelbehälter (51) für wasserfreie, ölbeladene Lösungsmittel-Flüssigkeit zugeführt wird.

2. Verfahren nach Anspruch 1, dadurch gekennzeichnet, daß aus dem Extraktor eine untere Schicht der Rest-Flüssigkeitsmenge aus sich gegenüber dem Wasseranteil absetzendem ölbeladenem Lösungsmittel über die Trennstufe in den Sammelbehälter für wasserfreie Lösungsmittel-Flüssigkeit und eine obere, weitestgehend lösungsmittelfreie Wasserschicht direkt in den abgetrenntes Wasser sammelnden Behälter geleitet wird.

3. Vorrichtung zum Durchführen des Verfahrens nach Anspruch 1 oder 2, mit einem Lösungsmittel- und einem Probenwasser-Dosierbehälter (4, 5), aus denen je eine Menge eines Lösungsmittels und eine Menge Probenwasser zu einem Extraktor (32) gefördert wird, das Öl aus dem Probenwasserstrom mit einem organischen Lösungsmittel extrahiert, das Wasser einer dem Extraktor entnommenen Meßprobenmenge in einem Trennfilter (43) abgetrennt und das ölbeladene Lösungsmittel in einem Analysator (45) analysiert wird, dadurch gekennzeichnet, daß das an dem Extraktor (32) angeschlossene, nachgeordnete Trennfilter (43) eine abgetrenntes Wasser führende und eine ölbeladene Lösungsmittel-Flüssigkeit führende Leitung aufweist, von denen die Lösungsmittel-Flüssigkeitsleitung (44, 50) in einen separaten Flüssigkeits-Sammelbehälter (51) mündet.

4. Vorrichtung nach Anspruch 3, dadurch gekennzeichnet, daß eine Entleerungsleitung (35) des Extraktors (32) vor dem Trennfilter (43) an eine mit dem Trennfilter (43) verbundene Meßleitung (34) des Extraktors (32) angeschlossen ist und die dem Filter (43) nachgeschaltete Leitung (44, 50) der ölbeladenen Lösungsmittel-Flüssigkeit einerseits zu dem Meßgerät (45) und andererseits mit einem sich einem in der Leitung (44) angeordneten Ablaufventil (46) anschließenden Leitungsfortsatz (50) in den Sammelbehälter (51) reicht.

0195124